# EUROPEAN PATENT APPLICATION

(11) **EP 0 970 702 A1**
(43) Date of publication of application: **12.01.2000**
(21) Application number: 97902681.2
(22) Date of filing: 14.02.1997
(51) Int. Cl.: A61K 35/84

(54) **BRAIN ACTIVATORS**

(30) Priority: 15.02.1996 JP 2801896
(71) Applicant: DAIICHI PHARMACEUTICAL CO., LTD., Chuo-ku, Tokyo 103 (JP)
(72) Inventor: SAITO, Hiroshi, Tokyo 150 (JP); NISHIYAMA, Nobuyoshi, Tokyo 174 (JP); ISHIHARA, Masanao Daiichi Pharmaceutical Co.,, Edogawa-ku Tokyo 134 (JP)
(74) Representative: Hartz, Nikolai F., Dr.
(86) International application number: JP9700390
(87) International publication number: WO9729764

(57) **Abstract**

The present invention relates to brain activators and anti-dementia drugs containing as the active ingredient(s) one or more saccharides selected from among low-molecular-weight pachyman originating in holen, lichenan, isolichenan, dextrans, curdlan, and pustulan; a derivative thereof; and a salt of any of these. Use of these drugs makes it possible to inhibit or treat lowering of learning and memory functions caused by various factors.

## Description

### Technical Field

The present invention relates to brain activators, more particularly to brain activators exhibiting excellent ameliorating effect for memory disorders (dementia) caused by senility and cerebral injury.

### Background Art

Amelioration of memory disorders (dementia) caused by senility and cerebral injury is an essential factor for ensuring quality of life (full and high-quality life in old age). However, therapeutic agents such as cerebral circulation and metabolism-ameliorating agents do not show sufficient effect to clearly ameliorate dementia in actual clinical situations.

Dementia is typically classified into (1) Alzheimer-type dementia, (2) cerebral vascular dementia, and (3) mixed-type dementia.

In (1) Alzheimer-type dementia, dementia is considered to be caused by accumulation of β-amyloid protein and τ-protein, insufficient concentration of active substances, and decrease of receptors in the brain of the patient. Examples of the insufficient and activity-decreased substances include neurotransmitters such as acetylcholine, dopamin, noradrenaline, serotonin, or GABA; and neuro-peptides such as vasopressin, substance P, or somatostatin. As therapeutic drugs for Alzheimer-type dementia, there are used cerebral metabolism-improving drugs which activate acetylcholine (such as idebenone, calcium hopantenate, or meclofenoxate hydrochloirde) and those which activate serotonin (such as bifemelane hydrochloride, indeloxazine hydrochloride, lisuride maleate, or aniracetam); intracerebral choline-agonist activators (such as acetylcholine precursors, physostigmine, or drugs similar to physostigmine); and serotonin-agonist activators such as acetylcholinesterase-inhibitors or muscarinic receptor agonists, serotonin reincorporation-inhibitors, and 5-HT₃ receptor antagonists. All of these are used to enhance activity of insufficient and activity-decreased substances and to increase concentration thereof.

Meanwhile, (2) cerebral vascular dementia is classified into three types: i) disorders induced by wide or multiple cerebral ischemia caused by cerebral infarction; or cerebral ischemia at the domain responsible for dementia (e.g., hippocampus, interior thalamus, or amygdaloid nucleus); ii) dementia caused by cerebral hemorrhage; and iii) dementia caused by subarachnnoid hemorrhage. All of these are disorders of brain functions caused by blocking or lowering (stagnating) blood flow. As in the case of Alzheimer-type dementia, there have been conducted a number of treatments by use of cerebral circulation- and metabolism-ameliorating drugs or cerebral metabolism-ameliorating drugs.

Briefly, with regard to treatment of the above-described Alzheimer-type dementia, there exist no drugs which suppress and prevent production and accumulation of β-amyloid protein and τ-protein, and there are exclusively selected drugs which are intended to increase the decreased concentration or activity of active substances. With regard to cerebral vascular dementia, treatment is assessed on the basis of functional recovery regarding neurotransmission at the domain of cerebral ischemia and peripheral sites thereof. Actually, the drugs are selected in accordance with intelligence evaluation tests of patients, evaluation of clinical conditions, and severity evaluation (by use of FAST staging), and choice of cerebral metabolism-ameliorating drugs or cerebral circulation-ameliorating drugs is determined in accordance with the type of dementia.

However, even the drugs developed in order to ameliorate intelligence function disorders, which are the principal symptoms of dementia, are hardly effective in ameliorating deterioration of intelligence functions, and they are actually effective only in ameliorating emotional disorders, volition disorders, problem behavior, etc. concomitant with dementia. Since "anti-dementia drugs" and "nootropic drugs" in their true sense cannot be obtained at present, it is considered that there are still no drugs which are worthy of the name "anti-dementia drugs" (Journal of Clinical Mental Medicine, special issue, "Psychopathy Today" 1995, from "Anti-dementia drugs").

In recent years, there has been elucidated action which increases neuro-synaptic transmission and plasticity related to memory and learning at the brain and potentiates and which maintains memory stimulation, called "Long Term Potentiation" (LTP). It is thereby proven that intense stimulation to the hippocampus and dentate gyrus in the brain relates to consolidating and preserving memory. Briefly, the phenomenon called Long Term Potentiation (LTP), in which local (the hippocampus and dentate gyrus) electric potential in the brain significantly increases 10 minutes after application of high-frequency tetanus stimulation in a short time to the brain (mainly to the hippocampus and dentate gyrus), is highly responsible for memory and learning. Measurement of the summed electric potential of LTP has revealed that the LTP is reinforced through LTP potentiation attributed to drugs. In the LTP potentiation, stimulation attributed to temporally input information is amplified and maintained in the memory field of the brain to thereby result in eternal storage, while weak stimulation is canceled to result in oblivion. This is considered to be partially involved in the basic memory process. Therefore, deterioration of learning and memory functions relates to deterioration of neuro-synaptic plasticity at the brain hippocampus and dentate gyrus. In other words, potentiation and persistence of LTP is considered to correlate to amelioration of memory functions of the brain.

Accordingly, an object of the present invention is to provide a pharmaceutical which inhibits or treats deterioration of learning and memory functions of the brain, i.e., to provide drugs which exhibit excellent potentiation and persistence action for the above-described LTP, which activate cerebral functions such as learning and memory functions, and which are effective for treatment of dementia.

### Disclosure of the Invention

The present inventors, focusing their attention on plant-derived substances from the aspect of safety and also in consideration of long-term administration to the aged, conducted screening of a variety of components in order to potentiate and maintain LTP, and found that specific saccharides exhibit excellent potentiation and persistence effect for LTP and are useful for brain activators and anti-dementia drugs. The present invention was accomplished based on this finding.

Accordingly, in one aspect of the present invention, there are provided brain activators containing as the active ingredient(s) one or more saccharides selected from among low-molecular-weight pachyman originating in hoelen, lichenan, isolichenan, dextrans, curdlan, and pustulan; a derivative thereof; and a salt of any one of these.

In another aspect of the present invention, there are provided anti-dementia drugs containing as the active ingredient(s) one or more saccharides selected from among low-molecular-weight pachyman originating in hoelen, lichenan, isolichenan, dextrans, curdlan, and pustulan; a derivative thereof; and a salt of any one of these.

In a further aspect of the present invention, there is provided a brain activator composition containing one or more saccharides selected from among low-molecular-weight pachyman originating in hoelen, lichenan, isolichenan, dextrans, curdlan, and pustulan; a derivative thereof; and a salt of any one of these; as well as a pharmaceutically acceptable carrier.

In a still further aspect of the present invention, there is provided an anti-dementia drug composition containing one or more saccharides selected from among low-molecular-weight pachyman originating in hoelen, lichenan, isolichenan, dextrans, curdlan, and pustulan; a derivative thereof; and a salt of any one of these; as well as a pharmaceutically acceptable carrier.

In a yet further aspect of the present invention, there is provided use of one or more saccharide(s) selected from among low-molecular-weight pachyman originating in hoelen, lichenan, isolichenan, dextrans, curdlan, and pustulan; a derivative thereof; and a salt of any one of these in order to manufacture brain activators.

In a still further aspect of the present invention, there is provided use of one or more saccharide(s) selected from among low-molecular-weight pachyman originating in hoelen, lichenan, isolichenan, dextrans, curdlan, and pustulan; a derivative thereof; and a salt of any one of these in order to manufacture anti-dementia drugs.

In a yet further aspect of the present invention, there is provided a method for activating the brain by administering to a patient an effective amount of one or more saccharide(s) selected from among low-molecular-weight pachyman originating in hoelen, lichenan, isolichenan, dextrans, curdlan, and pustulan; a derivative thereof; and a salt of any one of these.

In a still further aspect of the present invention, there is provided a method for treating dementia by administering to a patient an effective amount of one or more saccharide(s) selected from among low-molecular-weight pachyman originating in hoelen, lichenan, isolichenan, dextrans, curdlan, and pustulan; a derivative thereof; and a salt of any one of these.

### Brief Description of the Drawings

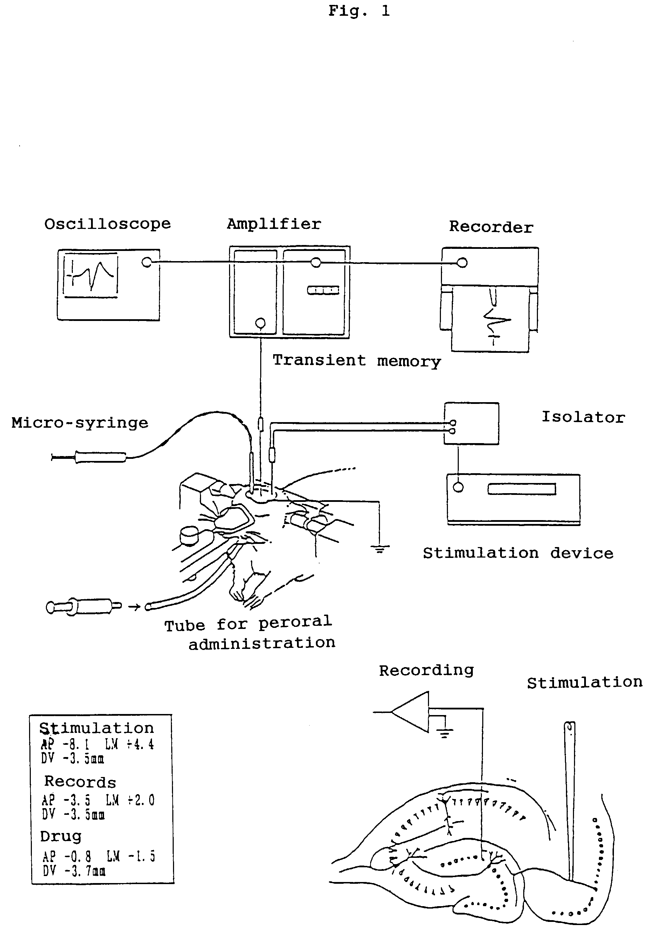

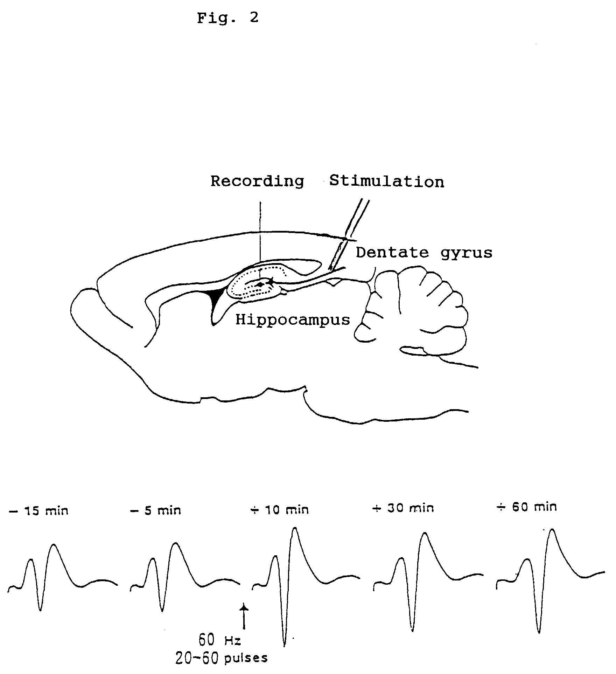

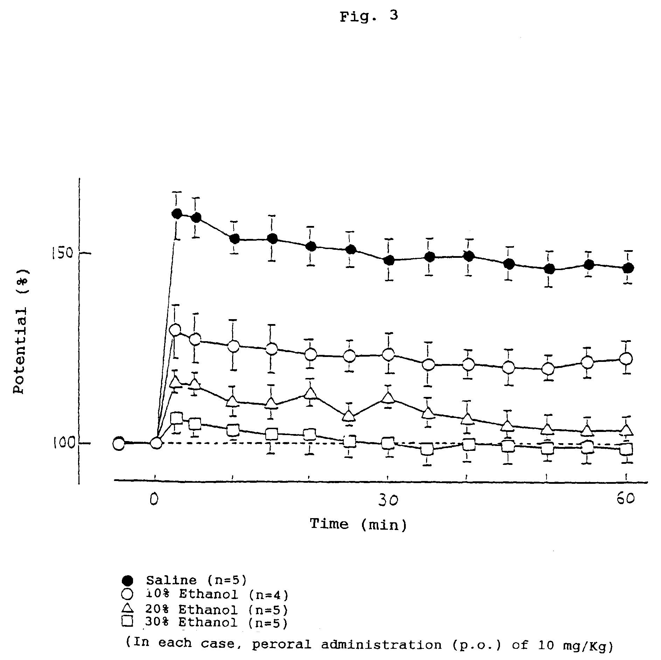

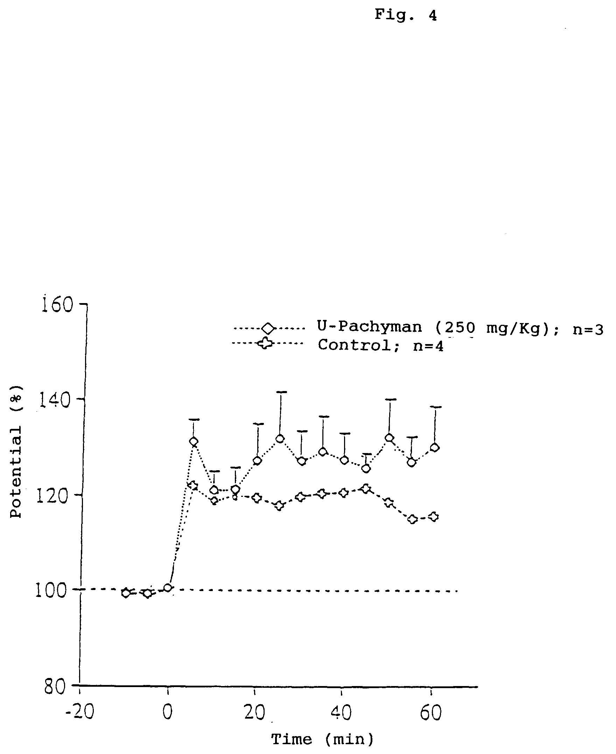

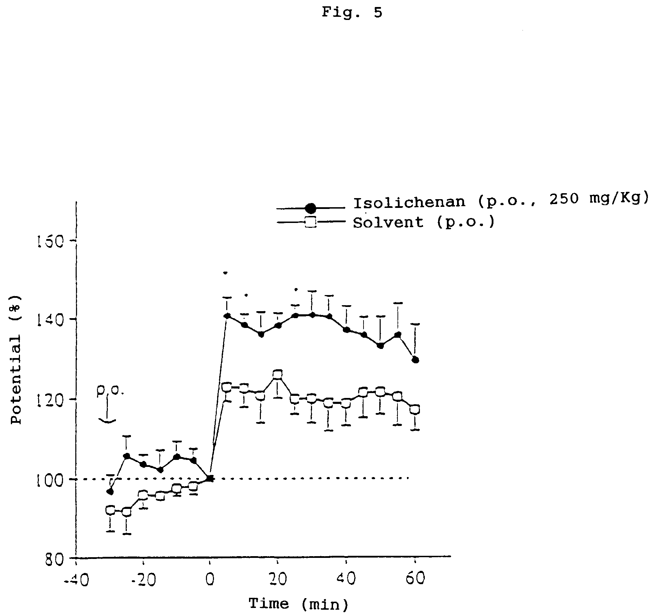

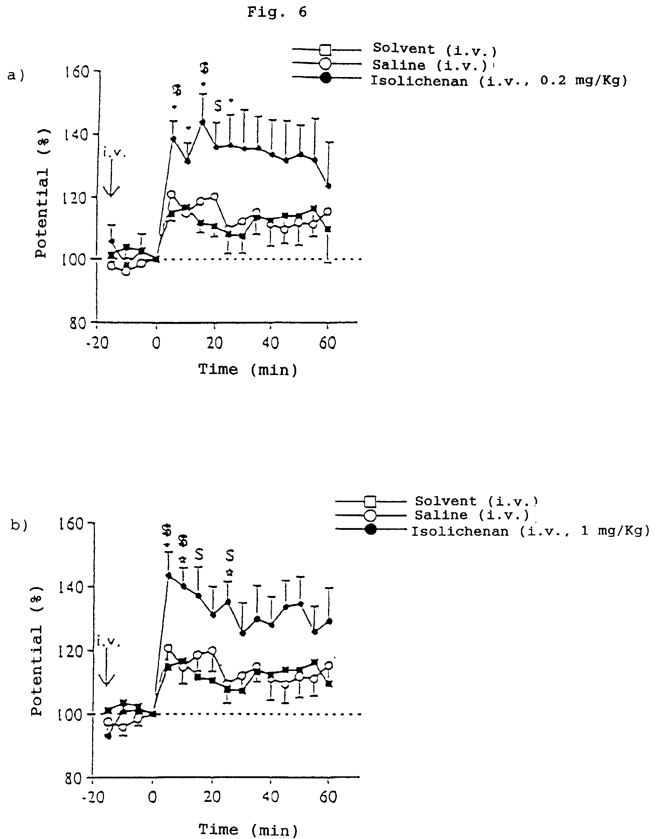

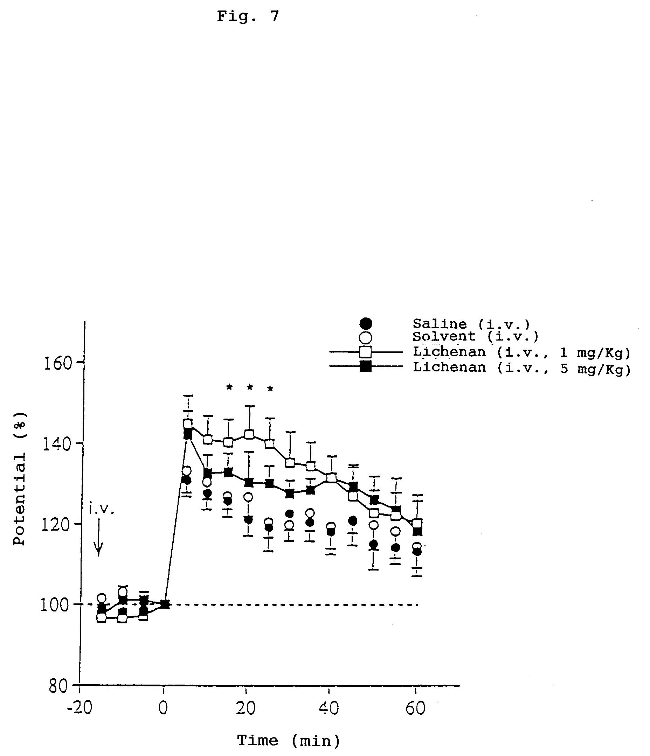

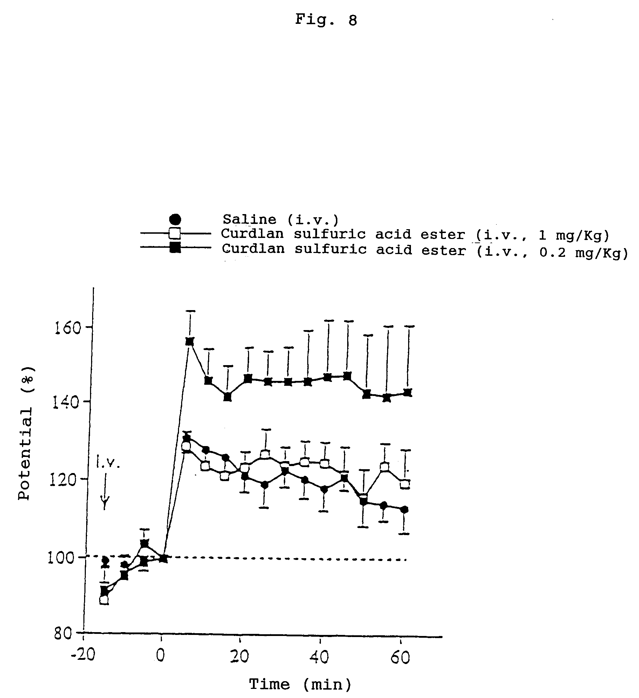

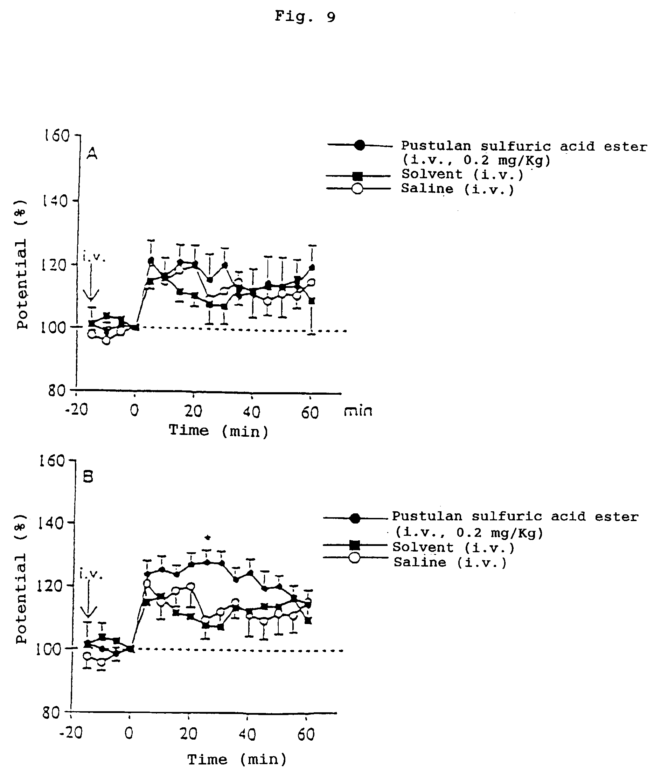

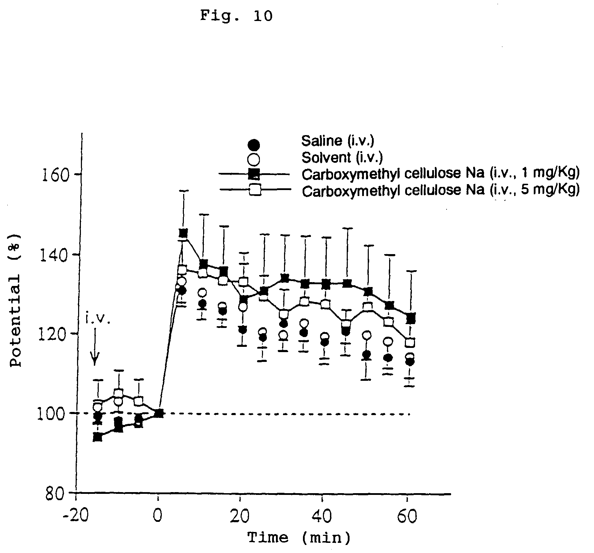

Fig. 1 shows an experimental apparatus for recording induced electric potential at the hippocampus of a rat under anesthesia. Fig. 2 shows an example of LTP records obtained at the perforated fiber-dentate gyrus synapse. Fig. 3 shows the effect of peroral administration of ethanol on LTP at the hippocampus of aged rats under anesthesia. Fig. 4 shows potentiation of LTP through peroral administration of urea-treated pachyman (U-pachyman). Fig. 5 shows potentiation of LTP through peroral administration of isolichenan. Fig. 6 shows potentiation of LTP through intravenous injection of isolichenan. Fig. 7 shows potentiation of LTP through intravenous injection of lichenan. Fig. 8 shows potentiation of LTP through intravenous injection of curdlan sulfuric acid ester. Fig. 9 shows potentiation of LTP through intravenous injection of pustulan sulfuric acid ester. Fig. 10 shows potentiation of LTP through intravenous injection of carboxymethyl cellulose.

### Best Mode for Carrying Out the Invention

The saccharides used in the drugs of the present invention are one or more saccharides selected from among low-molecular-weight pachyman originating in hoelen, lichenan, isolichenan, dextrans, curdlan, and pustulan; derivatives thereof; and salts of these. Examples of the derivatives include alkylated products, carboxyalkylated products, esterified products, and halogenated products. Examples of the alkylated products and carboxyalkylated products include the above-described saccharides in which a C₁₋₁₀ alkyl group or a C₂₋₁₀ carboxyalkyl group bonds to at least one hydroxyl group thereof. Examples of the esterified products include the above-described saccharides in which an acid such as a C₂₋₃₆ fatty acid, phosphoric acid, sulfonic acid, or sulfuric acid bonds to at least one hydroxyl group thereof. Examples of the halogenated products include the above-described saccharides in which at least one hydroxyl group thereof is substituted with a halogen atom such as chlorine or bromine. Of these derivatives, fatty acid-esterified products, carboxyalkylated products, and sulfuric acid-esterified products are preferred, with acetylated products, carboxymethylated products, and sulfuric acid-esterified products being particularly preferred.

Examples of the salts of the above-described saccharides include alkali metal salts such as sodium salts and potassium salts; alkaline earth metal salts such as calcium salts and magnesium salts; iron salts; carbonate salts; lactate salts; phosphate salts; sulfonate salts; and EDTA salts.

The low-molecular-weight pachyman originating in hoelen is a low-molecular-weight fraction of sclerotium polysaccharides of *Paria cocos* generated at the subterranean root of a pine tree by *Polyporaceae Poria cocos* Wolf, a kind of *Basidiomycetes*. The pachyman is a linear β-glucan [β(1→ 3, 1→6)-D-glucan] having an absorption band at 890 cm⁻¹ in the infrared absorption spectrum, and is represented by the following formula.

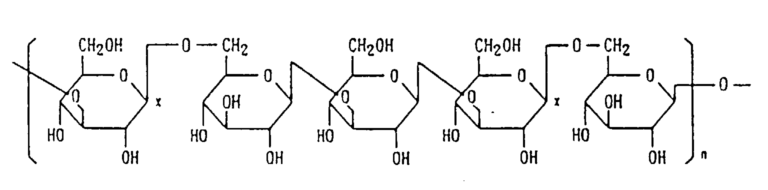

Low-molecular-weight pachyman, which is water-soluble, is obtained by, for example, extracting bark-removed sclerotium of hoelen with water, hot water, or an aqueous alkaline solution (*Yakugaku zassi*, 106(3), 199-205 (1986), *Nature*, Vol. 225, 943-944 (1970), *Carbohydrate Research*, 87, 161-163 (1980)).

Low-molecular-weight pachyman, sulfuric acid esters of pachyman, and carboxymethylated pachyman preferably have a molecular weight of 600-18,000.

Lichenan is a fraction of polysaccharides contained in an amount of approximately 10% in *Cetraria islandica* or *Rocella montagei*, a kind of *lichens*, and exhibits no coloring when iodine is added thereto. It is soluble to hot water and insoluble to cold water. The lichenan is a linear β-glucan [β(1→3, 1→4)-D-glucan] having an absorption band at 890 cm⁻¹ in the infrared absorption spectrum, and is represented by the following formula.

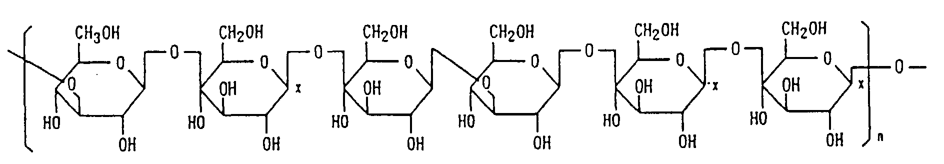

Lichenan may be obtained through, for example, extraction of *Cetraria islandica* with hot water to remove a fraction dissolved in cold water (N. B. Chanda *at at., J. Chem. Soc*., 1951 (1957), S. Peat *et at., J. Chem. Soc*, 3916 (1957)).

Lichenan, sulfuric acid esters of lichenan, acetylated lichenan, and triacetylated lichenan preferably have a molecular weight of 11,000-40,000.

Isolichenan, like lichenan, is a fraction of polysaccharides contained in *Cetraria islandica* or *Rocella montagei*, but exhibits coloring when iodine is added thereto. It has resistance to β-amylase and is soluble to cold water. Isolichenan is a linear α-glucan [α(1→3, 1→4)-D-glucan] having an absorption band at 840 cm⁻¹ in the infrared absorption spectrum, and is represented by the following formula.

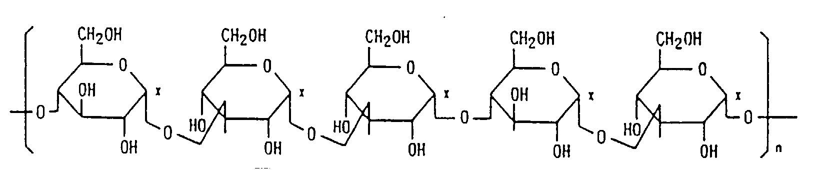

Isolichenan may be obtained from, for example, a fraction dissolved in cold water at the above-described recovery step of lichenan (the above-described literature).

Isolichenan and sulfuric acid esters thereof preferably have a molecular weight of 7,200-15,000.

Dextrans are formation/decomposition products of bacteria or yeast, and are linear or branched α-glucans [α(1 →3, 1→6)-D-glucan or α(1→6)-D-glucan] having an absorption band at 840 cm⁻¹ in the infrared absorption spectrum, and are represented by the following formula.

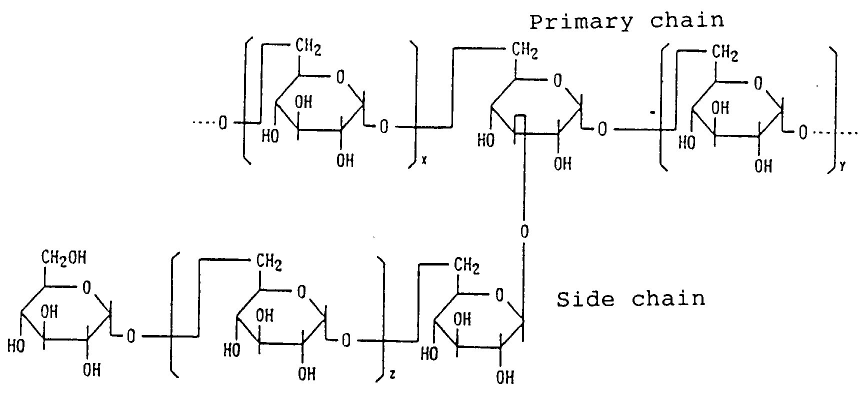

Dextrans may be obtained through, for example, a recovery method from a variety of cultured bacteria or an enzymatic synthesis by use of dextran sucrase (A. Jeanes, "Methods in Carbohydrate Chemistry" (Academic Press, New York), Vol. 5, p118, 127 (1965)).

Dextran and dextran sulfuric acid preferably have a molecular weight of 32,000-120,000.

Curdlan is a fraction of saccharides obtained from *Alcaligenes faecalis* var. *myxogenes* 10C3K and is a linear β-glucans [β(1→3)-D-glucan] having an absorption band at 890 cm⁻¹ in the infrared absorption spectrum, and is represented by the following formula.

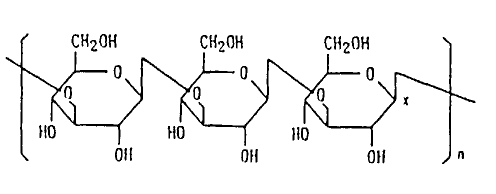

Curdlan may be obtained through, for example, a known recovery method from cultured *Alcaligenes faecalis* var. *myxogenes* 10C3K (T. Harada *et al. Arch. Biochem. Biophys*., Vol. 124, 292 (1968), H. Saito *et al., Agr. Biol. Chem*., Vol. 32, 1261 (1968)).

Curdlan, sulfuric acid esters of curdlan, and carboxymethylated curdlan preferably have a molecular weight of 10,000-90,000.

Pustulan is a fraction of saccharides obtained from *Umbilicura pustulata* (*Lasallia pustulata*) or *Umbilicura esculenta* (*Gyrophora esculenta*), which belongs to IWADAKE *Lichen*, and is a linear β-glucans [β(1→6)-D-glucan] having an absorption band at 890 cm⁻¹ in the infrared absorption spectrum, and is represented by the following formula.

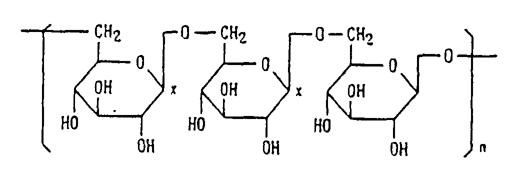

Pustulan may be obtained through, for example, the method described in *Chem. Pharm. Bull*. 16 (12), 2362-2369 (1968).

Pustulan and sulfuric acid esters thereof preferably have a molecular weight of 10,000-22,000.

The above-described saccharides may be incorporated into the drugs of the present invention singly or in combination of two or more species.

As mentioned in Examples below, the drugs of the present invention exhibit an excellent potentiation and persistence action for an LTP action, and are therefore useful as brain activators and anti-dementia drugs. In the present invention, a brain activator refers to a drug which enhances synaptic plasticity of the hippocampus and dentate gyrus of the brain and synaptic conductivity and potentiates an LTP (Long Term Potentiation) action against deterioration of memory, remembering, and learning functions of the brain caused by brain injuries, drugs (e.g., alcohol), etc., and is administered perorally or parenterally (e.g., through intravenous injection) to thereby inhibit or treat deterioration of learning and memory functions and regain quality of life. An anti-dementia drug refers to a drug classified as a brain activator that is applied for a case in which memory, remembering, and learning functions of the brain are deteriorated by dementia. More specifically, the anti-dementia drug may be used as a memory disorder-ameliorating agent or an inhibitor against deterioration of learning and memory functions in Alzheimer-type dementia, cerebral vascular dementia, and mixed-type dementia.

The drugs of the present invention may be administrated perorally and parenterally, and peroral administration and intravenous injection are particularly preferred. Examples of the form thereof include tablets, capsules, granules, syrup, and injections. Additives such as excipients, binders, disintegrators, and solubilizers may be incorporated for preparing the drugs.

The dose of the drugs of the present invention is typically 1-1500 mg/day per adult, with 50-1000 mg for peroral administration and 1.2-100 mg for parenteral administration being preferred. The drugs may be administrated in a single dose, continuously, or in a divided manner, preferably through division into 2-3 times per day.

### Examples

The present invention will next be described in detail by way of examples, which should not be construed as limiting the invention.

### Reference Example 1: Model of memory disorder caused by ethanol and test through LTP measurement

### (1) Animals

### Wistar rats (8 weeks old, male)

### (2) Method

Ethanol, which is known to induce memory disorder, was orally administrated to rats. There was conducted an investigation as to whether or not there occurred lowering of long term potentiation (LTP) of summed electric potential at the hippocampus and dentate gyrus.

Groups of rats suited for the purpose of the test were anesthetized with urethane-chloralose, and each rat was fixed in a brain localization fixer. A polyethylene tube connected to a syringe was inserted into the stomach of the rat in advance. An injection needle of 25-gauge was employed for intravenous injection and an area near the femoral vein was cleaned with alcohol-impregnated cotton pieces.

After the above preparation, a hole was made in the cranial bone by use of the Bregma as the point of reference, and a bipolar stimulating electrode and a monopolar recording electrode were inserted to the cortex in the olfactory bulb and the hippocampus and dentate gyrus, respectively.

Tetanus stimulation (60-100 Hz, 60-100 pulses) was applied to perforated fibers by application of a single square wave (0.08 msec) every 30 seconds and the induction electric potential was extracellularly recorded from granule cells of the dentate gyrus.

The intensity of the tetanus stimulation was selected to a level where 50% of the maximum reaction occurs. The summed induced electric potential having a constant intensity of 100-200% was confirmed to have been recorded for at least 20 minutes (see Figs. 1 to 3).

### (3) Results

In reference to induction of memory disorders, peroral administration of 10%-40% ethanol in a dose of 10 ml/kg suppressed a post-tetanic LTP (long term potentiation) phenomenon, and no change in the pre-tetanic induction electric potential was observed. Dose dependency of peroral administration of ethanol on LTP-suppressing action was proven. The results indicate that memory disorder action imparted by ethanol is identified through lowering of LTP as an index (see Fig. 3, upper).

### Preparation Example

(1) Low-molecular-weight pachyman having a molecular weight of 600-10,000 was obtained through extraction of hoelen with hot water and lyophilization.
(2) *Lichens* (*Umbilicaria pustulata*) was extracted with water. To the extraction residue, ethanol was added and the mixture was filtered to obtain a fraction, which was extracted with hot water. After filtration, acetone was added to the resultant residue and the mixture was filtered to thereby obtain pustulan having a molecular weight of 18,000-20,000. The obtained pustulan was esterified with sulfuric acid to thereby obtain sulfated pustulan having a molecular weight of 10,000-20,000.

### Example 1: Measurement of LTP at the hippocampus and dentate gyrus and administration of drugs

### (1) Animals

### Wistar rats (8 weeks old, male)

### (2) Method

Effect of drugs on LTP was investigated after confirmation of stable recording of the induction electric potential in a manner similar to that of Reference Example 1.

In the process of measurement of LTP in the presence of drugs, the drug solutions were administrated 30 minutes before measurement in the case of peroral administration and 15 minutes before measurement in the case of intravenous injection. Then, tetanus stimulation (60 Hz, 20 pulses) was applied to the rat brain (mainly the hippocampus and dentate gyrus) and there was started extracellullar measurement of the electric potential induced from granule cells of the hippocampus and dentate gyrus. Subsequently, the intensity of summed induction electric potential (degree of potentiation of the electric potential) was regulated between 100% to 200%, and was monitored for 0-60 minutes after tetanus stimulation. To facilitate observation of the intensity of the potentiation caused by the drugs, the intensity of the stimulation was reduced so that the summed electric potential had a value of 100% to that of a saline group used as a control.

Comparison was performed by use of a drug-free solvent (3-4% DMSO or saline) solution.

### (3) Evaluation

++: Statistically significant difference between the tested drug and the control was very clear.
+: Statistically significant difference between the tested drug and the control was clear.
-: No statistically significant difference, or similar effect was found between the tested drug and the control.

### (4) Results

The complete results are shown in Tables 1 to 2 and Figs. 4 to 10.

**Table 1**

| Results of peroral administration | |
|---|---|
| Drug | Peroral administration (250 mg/kg) |
| Pachyman (low-mol. wt.)^{*1} | ++ |
| Urea-treated pachyman*² | - |
| Isolichenan (PC-2)^{*3} | ++ |
| Curdlan^{*4} | ++ |

| | |
|---|---|
| *1 : Obtained in accordance with Manufacture Example (1). | |
| *2 : Powder (molecular weight 10,000-100,000) obtained by extracting hoelen with hot water; separating to an ether-acetone-water mixture; fractionating an ethanol-dissolved solution with 8 M urea; treating excess urea (desalting and removing urea); and drying under vacuum. | |
| *3 : Molecular weight 7,200-9,000 (av. mol. wt. 8,100). | |
| *4 : Average molecular weight 90,000 (av. polymerization degree 500) (product of Sigma Co.; CURDLAN, C7821). | |

**Table 2**

| Results of intravenous injection | | | |
|---|---|---|---|
| Drug | Intravenous injection (/kg) | | |
| | 0.2 mg | 0.5 mg | 1.0 mg |
| Pachyman (low-molecular wt.)^{*1} | - | - | ++ |
| Urea-treated pachyman^{*2} | - | - | - |
| Isolichenan^{*3} | ++ | ++ | ++ |
| Dextran^{*5} | - | - | ++ |
| Lichenan^{*6} | - | - | ++ |
| Curdlan sulfuric acid ester^{*7} | - | | + |
| Pustulan sulfuric acid ester^{*8} | - | | + |
| Carboxymethyl cellulose^{*9} | - | | - |
| Starch (AS-30)^{*10} | - | | - |

| | | | |
|---|---|---|---|
| *1 : Obtained in accordance with Manufacture Example (1). | | | |
| *2 : Powder (molecular weight 10,000-100,000) obtained by extracting hoelen with hot water; separating to an ether-acetone-water mixture; fractionating an ethanol-dissolved solution with 8 M urea; treating excess urea (desalting and removing urea); and drying under vacuum. | | | |
| *3 : Molecular weight 7,200-9,000 (av. mol. wt. 8,100). | | | |
| *5 : Dextran 40 (mol. wt. 32,000-45,000) or Dextran 70 (mol. wt. 68,000-90,000) (product of Pharmacia Co.). The same results were obtained for the two types of dextran. | | | |
| *6 : Molecular weight 3,600-5,400 (av. polymerization degree D.P. 100-200, product of Merck Co.). | | | |
| *7 : Average molecular weight 10,000 (Ajinomoto Co., Inc.) | | | |
| *8 : Molecular weight 10,000-20,000 (obtained in accordance with Manufacture Example (2)). | | | |
| *9 : 033-15221 CM-Cellulose Type II (product of Wako Pure Chemical Industries, Ltd.). | | | |
| *10 : Starch (AS-30) (product of Ajinoki Co.). | | | |

As clearly indicated by the results shown in Tables 1 to 2 and Figs. 4 to 10, the drugs of the present invention exhibit excellent potentiation and persistence action for an LTP action, which other saccharides do not exhibit.

### Industrial Applicability

The drugs of the present invention containing a specific saccharide as the active ingredient exhibit an excellent potentiation and persistence action for an LTP action, and are useful for inhibition or treatment of memory disorders in Alzheimer-type dementia, cerebral vascular dementia, and mixed-type dementia.

## Claims

1. A brain activator containing as active ingredient(s) one or more saccharides selected from among low-molecular-weight pachyman originating in hoelen, lichenan, isolichenan, dextrans, curdlan, and pustulan; a derivative thereof; and a salt of any one of these.

2. The brain activator according to claim 1, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

3. An anti-dementia drug containing as active ingredient(s) one or more saccharides selected from among low-molecular-weight pachyman originating in hoelen, lichenan, isolichenan, dextrans, curdlan, and pustulan; a derivative thereof; and a salt of any one of these.

4. The anti-dementia drug according to claim 3, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

5. A brain activator composition containing one or more saccharides selected from among low-molecular-weight pachyman originating in hoelen, lichenan, isolichenan, dextrans, curdlan, and pustulan; a derivative thereof; and a salt of any one of these; as well as a pharmaceutically acceptable carrier.

6. The brain activator composition according to claim 5, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

7. An anti-dementia drug composition containing as active ingredient(s) one or more saccharides selected from among low-molecular-weight pachyman originating in hoelen, lichenan, isolichenan, dextrans, curdlan, and pustulan; a derivative thereof; and a salt of any one of these; as well as a pharmaceutically acceptable carrier.

8. The anti-dementia drug composition according to claim 7, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

9. Use of one or more saccharide(s) selected from among low-molecular-weight pachyman originating in hoelen, lichenan, isolichenan, dextrans, curdlan, and pustulan; a derivative thereof; and a salt of any one of these in order to manufacture a brain activator.

10. The use according to claim 9, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

11. Use of one or more saccharide(s) selected from among low-molecular-weight pachyman originating in hoelen, lichenan, isolichenan, dextrans, curdlan, and pustulan; a derivative thereof; and a salt of any one of these for the manufacture of an anti-dementia drug.

12. The use according to claim 11, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

13. A method for activating the brain by administering to a patient an effective amount of one or more saccharide(s) selected from among low-molecular-weight pachyman originating in hoelen, lichenan, isolichenan, dextrans, curdlan, and pustulan; a derivative thereof; and a salt of any one of these.

14. The method for activating the brain according to claim 13, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.

15. A method for treating dementia by administering to an patient an effective amount of one or more saccharide(s) selected from among low-molecular-weight pachyman originating in hoelen, lichenan, isolichenan, dextrans, curdlan, and pustulan; a derivative thereof; and a salt of any one of these.

16. The method for treating dementia according to claim 15, wherein the low-molecular-weight pachyman originating in hoelen, the derivative of pachyman, and the salt of pachyman or derivative of pachyman has a molecular weight ranging from 600 to 18,000; the lichenan, the derivative of lichenan, or the salt of lichenan or derivative of lichenan has a molecular weight ranging from 11,000 to 40,000; the isolichenan, the derivative of isolichenan, or the salt of isolichenan or derivative of isolichenan has a molecular weight ranging from 7,200 to 15,000; the dextrans, the derivatives of dextrans, or the salts of dextrans or derivatives of dextrans have a molecular weight ranging from 32,000 to 120,000; curdlan, the derivative of curdran, or the salt of curdlan or derivative of curdlan has a molecular weight ranging from 10,000 to 90,000; and the pustulan, the derivative of pustulan, or the salt of pustulan or derivative of pustulan has a molecular weight ranging from 10,000 to 22,000.
